# NEUE EUROPÄISCHE PATENTSCHRIFT

(11) **EP 2 107 884 B2**
(45) Veröffentlichungstag und Bekanntmachung des Hinweises auf die Entscheidung über den Einspruch: **29.09.2021**
(45) Hinweis auf die Patenterteilung: 22.12.2010
(21) Anmeldenummer: 07819550.0
(22) Anmeldetag: 02.11.2007
(51) Int. Cl.: A61B 3/12

(54) **VERFAHREN UND GERÄT ZUR NETZHAUTDIAGNOSTIK**
METHOD AND APPARATUS FOR RETINAL DIAGNOSIS
PROCEDE ET APPAREIL DE DIAGNOSTIC DE LA RETINE

(30) Priorität: 02.11.2006 DE 102006052149
(43) Veröffentlichungstag der Anmeldung: 14.10.2009
(73) Patentinhaber: Heidelberg Engineering GmbH, 69115 Heidelberg (DE)
(72) Erfinder: ZINSER, Gerhard, 67346 Speyer (DE); ENGELHARDT, Ralf, 23568 Lübeck (DE); FISCHER, Jörg, 69221 Dossenheim (DE); MÜLLER, Frank Karlheinz, 67346 Speyer (DE); OTTO, Tilman, 67117 Limburgerhof (DE)
(74) Vertreter: Wesch, Arno
(86) Internationale Anmeldenummer: PCT/EP2007/009526
(87) Internationale Veröffentlichungsnummer: WO 2008/052793

(56) Entgegenhaltungen:
- EP-A- 1 836 952
- WO-A-2006/058735
- WO-A-2007/061769
- US-A1- 2004 036 838
- YANNUZZI L A ET AL: "Ophthalmic fundus imaging: today and beyond" AMERICAN JOURNAL OF OPHTHALMOLOGY, OPHTHALMIC PUBL., CHICAGO, IL,, US, Bd. 137, Nr. 3, März 2004 (2004-03), Seiten 511-524, XP004755596 ISSN: 0002-9394

## Beschreibung

Die Erfindung bezieht sich auf ein Verfahren zur Netzhautdiagnostik gemäß den im Oberbegriff des Patentanspruchs 1 angegebenen Merkmalen. Ferner bezieht sich die Erfindung auf eine Vorrichtung zur Durchführung des Verfahrens.

Aus der US 2004/036 838 A1 ist ein derartiges Verfahren zur Untersuchung der Netzhaut bekannt, wobei Daten und Flächenbilder der Netzhaut mittels einer ersten Systemkomponente, welche eine Abtasteinheit enthält, erzeugt werden. Ferner werden mittels eines OCT-Interferometers aus der Netzhaut zweite Daten und zweite Tiefenschnittbilder erzeugt, deren Position in dem genannten Flächenbild bekannt ist und/oder aus dem Flächenbild vorgegeben wird. Es ist nur eine einzige Abtasteinheit vorgesehen, welche mittels eines Sägezahn-Generators angesteuert wird. Die Strahlung sowohl für die Flächenbilder als auch die Tiefenschnittbilder gelangt über eine Einheit zur Fokuseinheit auf die gleiche gemeinsame Abtasteinheit. Eine voneinander unabhängige Erzeugung und Manipulation der Flächenbilder einerseits und der Tiefenschnittbilder andererseits ist nicht ohne Weiteres möglich.

Weiterhin ist aus der Druckschrift "YANNUZZI L A ET AL: "Ophthalmic fundus imaging: today and beyond" AMERICAN JOURNAL OF OPHTHALMOLOGY, OPHFTHALMIC PUBL., CHICAGO, IL., Bd. 137, Nr. 3, März 2004 (2004-03), Seiten 511-524, XP004755596 ISSN: 0002-9394" ein integriertes System bekannt, welches ein OCT-Ophthalmoskop und ein Angiografie-Gerät umfasst, wobei die gleichzeitige und korrelierte Darstellung von Oberfiächenbildern und Tiefenschnittbüdern der Netzhaut ermöglicht wird. Hinweise zur Vorgabe der Ausrichtung des Tiefenschnittbildes anhand des Flächenbildes oder die Stabilisierung des Tiefenschnittbildes sind diesem Dokument nicht zu entnehmen.

Ferner ist aus der EP 1 487 322 B1 ein Verfahren bekannt, welches zur Untersuchung von Gefäßen der Netzhaut eines Auges dient und die Bestimmung der Gefäßwanddicke ermöglicht. Hierbei wird mittels Laserabtastung zum einen der äußere Durchmesser und zum anderen der innere Durchmesser des Gefäßes und schließlich aus den derart ermittelten Daten die Wanddicke des Gefäßes bestimmt. Die Bestimmung des äußeren Gefäßdurchmessers erfolgt aus den Daten eines Reflexionsbildes und der innere Durchmesser entsprechend dem Durchmesser der bewegten Blutsäule aus den Daten eines Laserdopplerbildes. Eine umfassende Untersuchung oder Diagnostik der Netzhaut ist hierbei nicht ohne Weiteres möglich. Die Laserscanningtechnik wird zur Erzeugung von Reflexionsbildern, Angiographiebildern und Autofluoreszbildern der Netzhaut eingesetzt. Dabei wird die Netzhaut mit einem Laserstrahl bestimmter Wellenlänge in einem zweidimensionalen Feld Punkt für Punkt abgetastet, und es entstehen kontinuierlich laufende (Live) Bilder oder Referenzbilder, wobei 10 bis 50 Bilder pro Sekunde typisch sind. Die Angiographie, insbesondere in Form der Fluoreszein-Angiographie (FA) und der Indozyaningrün-Angiographie (ICGA), ist ein wichtiges diagnostisches Verfahren, wobei im Wesentlichen zweidimensionale Bilder der Netzhautoberfläche und ggfs. tieferliegender Schichten der Netzhaut erzeugt werden durch punktweises Beleuchten und Rastern bzw. Abtasten. Ferner können derartige Flächenbilder der Netzhaut durch flächige Beleuchtung und Erfassung eines geeignetes Bildsensors, wie beispielsweise einer CCD-Kamera (Charge Coupled Device) oder einer Fundus-Kamera erzeugt werden. Weiterhin gelangt zur Netzhautdiagnostik die Spektraldomänen Optische Kohärenz Tomographie (OCT) zum Einsatz, welche zweidimensionale Schnittbilder im Wesentlichen senkrecht zur Netzhautoberfläche liefert, sogenannte B-Scans, welche linienförmig zusammengesetzt sind aus in die Tiefe gehenden A-Scans. Zur Erzeugung der erstgenannten Flächenbilder und der Tiefenschnittbilder sind zwei unterschiedliche Geräte erforderlich, wodurch ein erheblicher Aufwand bedingt ist. Die Untersuchung bzw. Diagnostik der Netzhaut des Auges stellt im Hinblick auf die verschiedenen bekannten Verfahren hohe Anforderungen an einen Untersucher aber auch an einen Patienten und ist zudem zeitaufwändig, zumal die Untersuchungen mittels verschiedener Geräte nacheinander durchzuführen sind.

Hiervon ausgehend liegt der Erfindung die Aufgabe zugrunde, ein Verfahren vorzuschlagen, welches in einfacher Weise eine umfassende Untersuchung bzw. Diagnostik der Netzhaut des Auges ermöglicht. Das Verfahren soll problemlos und funktionssicher durchführbar sein und mit einem geringen Aufwand zuverlässige Ergebnisse liefern sowie die Diagnose für den Untersucher erleichtern. Das Gerät zur Durchführung des Verfahrens soll einen einfachen und/oder funktionssicheren Aufbau aufweisen, einen geringen Bedienungsaufwand erfordern und eine optimierte und/oder umfassende Netzhautdiagnostik ermöglichen.

Die Lösung dieser Aufgabe erfolgt hinsichtlich des Verfahrens gemäß den im Patentanspruch 1 angegebenen Merkmalen. Hinsichtlich des Gerätes erfolgt die Lösung gemäß den Merkmalen des auf das Gerät gerichteten Patentanspruchs.

Das erfindungsgemäß vorgeschlagene Verfahren sowie das zu dessen Durchführung vorgeschlagene Gerät ermöglichen die Durchführung von umfassenden Untersuchungen der Netzhaut aufgrund der Erzeugung von Flächenbildern in Kombination mit Tiefenschnittbildern der Netzhaut, wobei gleichzeitig oder nacheinander oder simultan ein Flächenbild und ein Tiefenschnittbild mittels einer oder mehrerer Anzeigeeinheiten, wie Monitoren oder Displays, dargestellt werden. In bevorzugter Weise kann ein Untersucher in dem Flächenbild unter Berücksichtigung eines in diesem dargestellten Bereich des Interesses, insbesondere einer sichtbaren Pathologie, eine beliebige Position und/oder Richtung des Tiefenschnittbilds auswählen und/oder vorgeben. Das Verfahren und das Gerät gelangen in bevorzugter Weise zur umfassenden Netzhautdiagnostik zum Einsatz.

Die Flächenbilder sind bevorzugt zweidimensionale Bilder von zumindest Teilbereichen der Netzhautoberfläche und/oder zu dieser zumindest näherungsweise parallelen Flächen der Netzhaut. Die Flächenbilder werden bevorzugt durch Angiographie erzeugt, doch können im Rahmen der Erfindung statt dessen auch Reflexionsbilder beliebiger Wellenlänge oder Autofluoreszenzbilder oder durch flächige Beleuchtung und mittels Kamera, wie CCD-Sensor, erfaßt Bilder verwendet und dargestellt werden. Die Tiefenschnittbilder aus der Netzhaut werden bevorzugt durch Optische Kohärenz Tomographie (OCT) erzeugt, doch können im Rahmen der Erfindung auch andere Verfahren, wie Ultraschall, zum Einsatz gelangen.

Das erfindungsgemäße Gerät zeichnet sich durch die Integration und Kombination zweier unabhängiger Systemkomponenten aus, mittels welchen zum einen die Flächenbilder und zum anderen die Tiefenschnittbitder erzeugt werden. So enthält die eine Systemkomponente bevorzugt ein Laser-Scan-System mit unterschiedlichen Laserquelien zur Aufnahme von Reflexions- und Angiographiebildern, zwei oszillierende Spiegel, mit denen der fokussierte Laserstrahl sequentiell ein zweidimensionales Gebiet der Netzhaut abtastet, sowie einen Detektor, welcher die Menge des an jedem Punkt reflektierten bzw. emittierten Lichts mißt. Die andere Systemkomponente enthält bevorzugt ein Spektraldomänen-OCT-System mit breitbandiger Lichtquelle, wie zum Beispiel Superluminiszenzdiode, zwei weitere Scanspiegel zum Abtasten der Netzhaut und einen Spektrometer. Es können aber auch andere OCT Techniken verwendet werden. Die Strahlengänge der beiden Systemkomponenten werden an einer Stelle aufeinander abgestimmt, welche zwischen dem untersuchten Auge und den Scanspiegeln liegt, und zwar insbesondere durch eine optische Einheit und/oder optische Teil- und Abbildungseinheit. Mit dem Gerät erfolgt eine simultane und/oder gleichzeitige und/oder in vorgebbaren, bevorzugt kurzen Zeitabständen nacheinander durchgeführte Aufnahme eines der Flächenbilder, welches auch als Live-Referenzbild bezeichnet wird, mit einem der Tiefenschnittbilder, welches auch als OCT-Scan oder OCT-Live-Bild bezeichnet wird. Ein Referenzbild ist in bevorzugter Weise entweder ein Angiographiebild oder ein Reflexionsbild oder ein Autofluoreszenzbild. Diejenige Stelle auf der Netzhaut, an welcher das Tiefenschnittbild, insbesondere das OCT-Live-Bild, erzeugt wird sowie die Richtung des Tiefenschnittbilds kann vom Bediener bzw. Untersucher frei gewählt und vorgegeben werden, beispielsweise durch Mausklick in das Referenzbild.

Weiterhin wird gemäß dem Verfahren und/oder mit dem Gerät die Messung der Augenbewegung eines Patienten anhand der daraus resultierenden Bewegungen der im Referenzbild sichtbaren Strukturen durchgeführt. Es erfolgt die Stabilisierung des Tiefenschnittbilds, insbesondere des OCT-Live-Bilds, derart, dass die mittels des Referenzbilds gemessenen Augenbewegungen auf die Optik, z.B. Scanspiegel, der OCT-Systemkomponente rückgekoppelt werden, wobei diejenige Stelle der Netzhaut, an welcher das OCT-Live-Bild gewonnen wird, den Augenbewegungen folgt, und somit ein stabiles Bild immer an derselben Stelle aufgenommen und erzeugt wird. Des Weiteren kann eine Mittelung von mehreren aufeinanderfolgenden, wie vorstehend erläutert, stabilisierten OCT-Live-Bildern erfolgen, um das Signal-zu-Rausch-Verhältnis und somit die Qualität der OCT-Live-Bilder zu erhöhen. Ferner erfolgt in bevorzugter Weise die Darstellung der gemittelten Bilder, beispielsweise als gleitender Mittelwert, anstelle des normalen Tiefenschnittbildes, insbesondere des OCT-Live-Bildes.

Gemäß einer bevorzugten Ausgestaltung der Erfindung erfolgt während der Stabilisierung eine automatische Aufnahme von mehreren zueinander parallel verlaufenden aber leicht versetzten Tiefenschnittbildern und/oder OCT-Bildern. Jedes dieser Bilder kann ein zeitliches Mittel mehrerer aufeinanderfolgender Bilder an der gleichen Stelle sein und die Menge der derart gemittelten Bilder bildet zusammen ein dreidimensionales Tiefenschnittbild und/oder dreidimensionales OCT-Bild.

Gemäß einer besonderen Weiterbildung wird die genaue Position und Richtung im Referenzbild sowie das Referenzbild selbst eines während einer Untersuchung aufgenommenen Tiefenschnittbildes und/oder OCT-Bildes, insbesondere in einem geeigneten Speicher, gespeichert. Des Weiteren erfolgt mittels der so gespeicherten Information eine automatische Einstellung der Ablenk- und/oder Abtasteinheit, insbesondere der Scanspiegel der OCT-Systemkomponente, bei einer nachfolgenden Untersuchung bzw. Verlaufsuntersuchung derart, dass das Tiefenschnittbild und/oder OCT-Bild der Verlaufsuntersuchung an genau der gleichen Stelle aufgenommen wird, wie in der vorangegangenen Untersuchung. Somit kann in bevorzugter Weise ein direkter Vergleich der genannten Bilder auf Veränderungen der Netzhaut vorgenommen werden. Durch das erfindungsgemäße Verfahren und ebenso mit dem erfindungsgemäßen Gerät werden erste Daten und erste zweidimensionale Flächenbilder zumindest von Bereichen der Netzhaut erzeugt und ferner zweite Daten und zweidimensionale Tiefenschnittbilder aus der Netzhaut erzeugt, wobei die Position und die Ausrichtung des oder der Tiefenschnittbilder bezüglich der Netzhautoberfläche vorgegeben werden. Die Stelle oder der Bereich des Interesses, insbesondere der im zweidimensionalen Bild oder Flächenbild sichtbaren Pathologie, an welcher das Tiefenschnittbild erzeugt wird, und ferner dessen Ausrichtung wird somit anhand des Flächenbilds oder Referenzbilds ausgewählt und vorgegeben. Es ist ferner von besonderer Bedeutung, dass das erste Flächenbild und das Tiefenschnittbild gleichzeitig oder simultan oder auch in vorgebbaren oder vorgegebenen zeitlichen, bevorzugt kurzen Abständen dargestellt werden. Das Tiefenschnittbild wird durch Rückkopplung der Abtast- oder Ablenkeinheit derart stabilisiert, dass diejenige Stelle oder der Bereich der Netzhaut, an welcher das Tieienschnittbild gewonnen wird, den Augenbewegungen folgt und nachgeführt wird. Darüber hinaus wird in bevorzugter Weise aus mehreren aufeinanderfolgenden, insbesondere stabilisierten Tiefenschnittbildern eine Mittelung durchgeführt, wobei bevorzugt ferner das oder die gemittelten Tiefenschnittbilder insbesondere als gleitender Mittelwert dargestellt werden. Darüber hinaus erfolgt während der Stabilisierung eine automatische Aufnahme bzw. Erzeugung von mehreren im Wesentlichen parallel zueinander verlaufenden und in vorgegebenen kleinen Abständen versetzten Tiefenschnittbildern. Bevorzugt wird aus mehreren aufeinanderfolgenden, bevorzugt stabilisierten Schnittbildern ein dreidimensionales Bild erzeugt. Ferner werden die Position und/oder Ausrichtung des bevorzugt stabilisierten Tiefenschnittbilds und/oder des dreidimensionalen Tiefenschnittbilds und ferner bevorzugt auch das zugeordnete Flächen- oder Referenzbild gespeichert.

Das erfindungsgemäße Verfahren sowie das zur Durchführung vorgeschlagene Gerät sind die Kombination der Erzeugung von Flächenbildern der Netzhaut mit der Erzeugung von zweiten Daten und zweidimensionalen Tiefenschnittbildern aus der Netzhaut, wobei die Position der zweiten Daten in dem aufgenommenen zweidimensionalen Flächenbild der Netzhaut bekannt ist und/oder gespeichert wird. Die Position und die Ausrichtung eines oder mehreren Tiefenschnittbilder wird anhand des Flächenbildes kontrolliert, aktiv gesteuert und vorgegeben und so wird die Position und Ausrichtung eines oder mehrerer Tiefenschnittbilder anhand eines zuvor aufgenommenen zweidimensionalen Bildes oder Flächenbildes vorgegeben und aktiv kontrolliert. Ferner wird bevorzugt die Position und Ausrichtung eines oder mehrerer Tiefenschnittbilder anhand eines ersten aufgenommenen Flächenbildes vorgegeben und über eine Aktualisierung des Flächenbildes aktiv kontrolliert. Bevorzugt wird die Bewegung des aktualisierten Flächenbildes relativ zum ersten aufgenommenen Flächenbild bestimmt und die Position der Aufnahme oder Erzeugung eines oder mehrerer Tiefenschnittbilder an den vorgegebenen Orten oder Stellen der Netzhaut gesteuert. Es ist von besonderer Bedeutung, dass das erste Flächenbild oder das aktualisierte Flächenbild und das Tiefenschnittbild gleichzeitig und/oder simultan oder in vorgebbaren Zeitabständen mittels einer oder mehrerer Anzeigeeinheiten dargestellt werden. Es wird das Tiefenschnittbild durch Rückkopplung derart stabilisiert, dass diejenige Stelle der Netzhaut, an welcher das Flächenschnittbild gewonnen wird, den Augenbewegungen nachgeführt wird. Des Weiteren hat es sich als zweckmäßig erwiesen, dass aus mehreren aufeinanderfolgenden stabilisierten Tiefenschnittbildern eine Mittelung durchgeführt wird und/oder dass das oder die gemittelten Flächenschnittbilder insbesondere als gleitender Mittelwert dargestellt werden. In bevorzugter Weise erfolgt während der Stabilisierung eine automatische Aufnahme von mehreren parallel oder gemäß einem beliebigen und/oder vorgebbaren Muster verlaufenden und in vorgebbaren kleinen Abständen versetzten Tiefenschnittbildern. Aus diesen Tiefenschnittbildern und/oder aus mehreren aufeinanderfolgenden Tiefenschnittbildern wird in bevorzugter Weise ein dreidimensionales Tiefenschnittbild erzeugt.

Des Weiteren werden die Position des Tiefenschnittbilds im Referenzbild und das Referenzbild selbst gespeichert und es wird das Tiefenschnittbild in einer Verlaufsuntersuchung genau an der gleichen Stelle und/oder mit der gleichen Ausrichtung aufgenommen bzw. erzeugt wie bei einer vorangegangenen Untersuchung und zudem für weitere Untersuchungen gespeichert.

Das zweidimensionale Flächenbild wird zweckmäßig über reflektiertes oder reemittiertes Licht in einem oder mehreren ausgewählten Wellenlängenbereichen erzeugt oder es wird zur Aufnahme des Flächenbilds die Netzhaut mit Licht eines ausgewählten Wellenlängenbereichs oder mehrerer ausgewählter Wellenlängenbereiche beleuchtet. Ferner kann zur Aufnahme oder Erzeugung des zweidimensionalen Flächenbilds die Beleuchtung mittels eines Punktes erfolgen, welcher rasterförmig das vorgegebene Areal der Netzhaut abscannt oder die Beleuchtung mit einer Linie erfolgen, die über das vorgegebene Areal der Netzhaut geführt wird. Die Detektion kann mittels Punktdetektor oder mittels Zeilenkamera oder mittels Flächenkamera erfolgen. Die Aufnahme der OCT-Schnittbilder erfolgt vorteilhaft nach einem Fourier Domain OCT-Verfahren oder einem Time Domain OCT-Verfahren. Bevorzugt ist das zweidimensionale Flächenbild ein Angiographiebild. In einer besonderen Ausgestaltung der Erfindung werden ein erstes Angiographiebild und ein zweites zweidimensionales Flächenbild - z.B. ein Reflektionsbild - der Netzhaut gleichzeitig oder in einem sehr kurzen zeitlichen Abstand gleichzeitig aufgenommen, wobei die Kontrolle und Steuerung der Aufnahme der Tiefenschnittbilder anhand einer Aktualisierung des zweiten zweidimensionalen Flächenbildes der Netzhaut erfolgt. Im Falle einer späteren Verlaufsuntersuchung kann dann dieses zweite Flächenbild anstelle des ersten Flächensbilds zu Kontrolle, Steuerung und Stabilisierung der Tiefenschnittbilder verwendet werden. Von besonderer Bedeutung ist erfindungsgemäß die Erzeugung der Flächenbilder als Angiographiebilder in Kombination mit OCT-Schnittbildern und deren simultane Darstellung.

Die Erfindung wird nachfolgend anhand eines besonderen Ausführungsbeispiels näher erläutert, ohne dass insoweit eine Beschränkung erfolgt. Es zeigen:
- Fig. 1: ein Blockschaltbild,
- Fig. 2: die in einer Anzeigeeinheit nebeneinander dargestellten Bilder, und zwar links das Flachenbild als Angiographiebild und rechts das Tiefenschnitt- bild als OCT-Live-B-Scan-Bild.

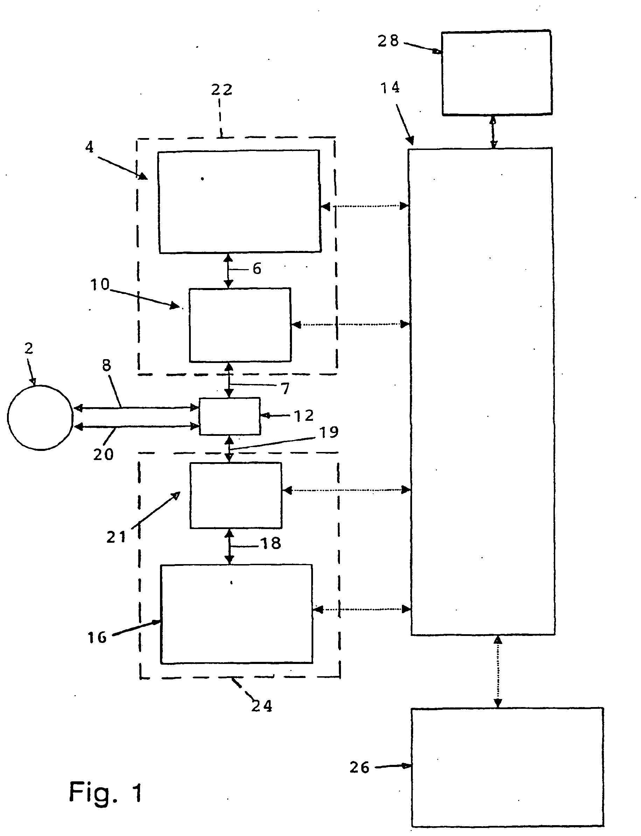

Gemäß Fig. 1 enthält das Gerät zur umfassenden Untersuchung der Netzhaut des schematisch dargestellten Auges 2 eine Bilderfassungseinheit 4 für erste zweidimensionale Bilder oder Flachenbilder der Netzhaut. Die Bilderfassungseinheit 4 enthält eine Beleuchtungseinheit und eine Messeinheit. Wie mittels der Pfeile 6, 7, 8 angedeutet, beleuchtet die Beleuchtungseinheit die Netzhaut, und zwar an einem Punkt, auf einer Linie oder auf einer Fläche. Hierbei gelangt das Licht über eine Abtaststeuereinheit 10 und eine optische Einheit 12 zur Netzhaut. Die Abtaststeuereinheit 10 enthält in bekannter Weise Scanspiegel oder dergleichen und wird ebenso wie die Bilderfassungseinheit 4 von einer Kontroll- und Verarbeitungseinheit 14 gesteuert. Eine Abtasteinheit 16 ist für die Erzeugung von Tiefenschnittbildern aus der Netzhaut vorgesehen. Das von der Netzhaut reflektierte und/oder reemittierte Licht gelangt gemäß den Pfeilen, 8, 7, 6 in die Bilderfassungseinheit 4, deren Messeinheit die Intensität des von einem Punkt der Netzhaut insgesamt reflektierten und/oder reemittierten Lichtes oder die Intensität des in einem ausgewählten Wellenlängenbereich reflektierten und/oder reemittierten Lichtes ist. Die Messeinheit ist entweder als ein Punktdetektor oder eine Zeilenkamera oder eine Flächenkamera ausgebildet.

Der OCT-Abtaststrahl für die OCT-Bildaufnahme tastet ein Areal auf der Netzhaut punktweise oder zeilenweise ab, wobei zu jedem Punkt ein Tiefenprofil, ein sogenannter OCT-A-Scan aufgenommen wird. Wie mit den Pfeilen 18, 19, 20 angedeutet, gelangt von der Abtasteinheit 16 das Licht über die OCT-Abtaststeuerung 20 und die optische Einheit 12 zur Netzhaut und zurück. Die OCT-Abtasteinheit 16 und die OCT-Abtaststeuerung 21 werden gleichfalls mittels der Kontroll- und Verarbeitungseinheit 14 gesteuert. Aufgrund der Kontrolle der Abtaststeuereinheit 10 und der OCT-Abtaststeuerung 21 sind die Koordinaten der OCT-Aufnahme und der Einheit 4 bekannt und können somit relativ zueinander gesteuert werden. Insbesondere kann der OCT-Abtaststrahl relativ auf feste Positionen und/oder Bereiche des Interesses gesteuert werden. Im Falle von Augenbewegungen, insbesondere in der Zeit zwischen einzelnen Aufnahmen der Bilderfassungseinheit 4, kann in bevorzugter Weise der OCT-Abtaststrahl nachgefahren werden.

Die Bilderfassungseinheit 4 und die Abtasteinheit 10 bilden eine erste Systemkomponente 22 und die OCT-Abtasteinheit 16 mit der OCT-Abtaststeuerung 21 bilden eine zweite Systemkomponente 24, deren Strahlengänge an einer Stelle und/oder mittels der optischen Einheit 12 vereinigt werden. Die optische Einheit 12 ist eine gemeinsame Teil- und Abbildungseinheit der beiden Systemkomponenten 22, 24 und/oder deren Strahlengänge. Wie ersichtlich, ist die optische Einheit 12 erfindungsgemäß in den Strahlengängen zwischen dem Auge 2 und einerseits der Abtaststeuereinheit 10 für die Flächenbilder und andererseits der Abtaststeuerung 21 für die Tiefenschnittbilder, insbesondere der OCT-Abtaststeuerung, angeordnet. An die Kontroll- und Verarbeitungseinheit 14 ist eine weitere Einheit 26 zur Bedienung und Auswertung angeschlossen, welche zudem vorteilhaft eine Datenbank und/oder Speicher und/oder eine CPU oder weitere Rechnermodule enthält. Schließlich ist an die Kontroll- und Verarbeitungseinheit 14 eine Anzeigeeinheit 28 angeschlossen, mittels welcher das Flächenbild, insbesondere Angiographiebild, sowie das OCT-Live-B-Scan-Bild dargestellt werden.

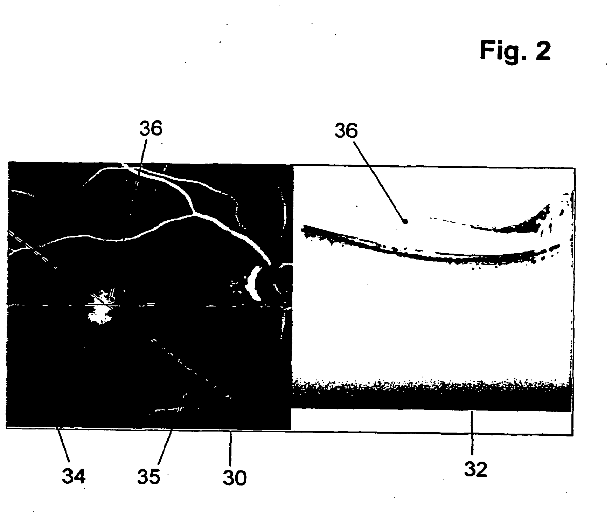

Fig. 2 zeigt die beiden mittels der Bildanzeigeeinheit dargestellten Bilder, und zwar links das Angiographiebild 30 und rechts das OCT-Live-B-Scan-Bild 32. Im Angiographiebild 30 ist mittels der Linie 34 diejenige Stelle, an welcher das Tiefenschnittbild bzw. OCT-Live-B-Scan-Bild aufgenommen ist, markiert. Die Linie 34 ist durch einen Bereich 36 gelegt und im rechten OCT-Live-B-Scan-Bild 32 sind weitere wichtige Einzelheiten dieses Bereiches 36 des Interesses für einen Untersucher klar erkennbar und bewertbar dargestellt. Die Positionierung der Linie 34 und deren Ausrichtung im Flächenbild 30 und somit bezüglich der Netzhaut kann der Bediener oder Untersucher problemlos nach seinen Erfahrungen oder Wünschen vorgeben, und nur beispielshaft ist eine weitere Linie 35 gestrichelt eingezeichnet mit geänderter Position und Ausrichtung, für welche ein entsprechend verändertes Tiefenschnittbild bzw. OCT-Live-B-Scan-Bild zur Darstellung gebracht werden kann.

### Bezugszeichen

- 2: Auge
- 4: Bilderfassungseinheit
- 6 - 8: Pfeil
- 10: Abtasteinheit
- 12: gemeinsame optische Einheit
- 14: Kontroll- und Verarbeitungseinheit
- 16: Abtaststeuereinheit
- 18 - 20: Pfeil
- 21: OCT-Abtaststeuerung
- 22: erste Systemkomponente
- 24: zweite Systemkomponente
- 26: weitere Einheit
- 28: Bild-Anzeigeeinheit
- 30: Flächenbild / Angiographiebild
- 32: Tiefenschnittbild / OCT-Live-B-Scan-Bild
- 34,35: Linie
- 36: Bereich des Interesses

## Patentansprüche

1. Verfahren zur Untersuchung der Netzhaut, wobei mittels einer ersten optischen Systemkomponente (22), welche eine erste Abtasteinheit (10) und eine Bilderfassungseinheit (4) enthält, erste Daten und Flächenbilder (30) der Netzhaut erzeugt werden, wobei ferner zweite Daten und zweidimensionale Tiefenschnittbilder (32) aus der Netzhaut erzeugt werden und die Position dieser zweiten Daten in dem aufgenommenen Flächenbild (30) der Netzhaut bekannt ist und/oder gemäß dem Flächenbild vorgegeben wird, wobei weiterhin mittels einer zweiten Systemkomponente (24), welche eine zweite Abtasteinheit (16) und eine Abtaststeuerung (21) enthält, die Tiefenschnittbilder (32) erzeugt werden, und auf dem Flächenbild (30) die Stelle (36) und die Ausrichtung (34, 35) des Tiefenschnittbildes (32) ausgewählt und vorgegeben wird,
**dadurch gekennzeichnet, dass** das Tiefenschnittbild (32) durch Rückkopplung der mittels des Flächenbilds (30) gemessenen Augenbewegungen auf die Abtasteinheit (16) der zweiten Systemkomponente (24) stabilisiert wird, wobei diejenige Stelle der Netzhaut, an welcher das Tiefenschnittbild gewonnen wird, den Augenbewegungen nachgeführt wird.

2. Verfahren nach Anspruch 1, **dadurch gekennzeichnet, dass** die Position und Ausrichtung eines oder mehrerer Tiefenschnittbilder (32) anhand des Flächenbildes (30) oder eines zuvor aufgenommenen Flächenbildes kontrolliert oder aktiv gesteuert oder vorgegeben wird.

3. Verfahren nach Anspruch 1 oder 2, **dadurch gekennzeichnet, dass** die Position und Ausrichtung eines oder mehrerer Tiefenschnittbilder (32) anhand eines ersten aufgenommenen Flächenbildes (30) vorgegeben und über eine Aktualisierung des Flächenbildes aktiv kontrolliert wird und/oder dass die Bewegung des aktualisierten Flächenbildes relativ zum ersten aufgenommenen Flächenbild bestimmt wird.

4. Verfahren nach einem der Ansprüche 1 bis 3, **dadurch gekennzeichnet, dass** das erste Flächenbild (30) oder das aktualisierte Flächenbild und das Tiefenschnittbild (32) gleichzeitig oder simultan oder in vorgebbaren, bevorzugt kleinen, Zeitabständen mittels einer oder mehrerer Anzeigeeinheiten (28) dargestellt werden.

5. Verfahren nach einem der Ansprüche 1 bis 4, **dadurch gekennzeichnet, dass** aus mehreren aufeinanderfolgenden, bevorzugt stabilisierten Tiefenschnittbildern, eine Mittelung durchgeführt wird und/oder dass das oder die gemittelten Tiefenschnittbilder insbesondere als gleitender Mittelswert dargestellt werden.

6. Verfahren nach einem der Ansprüche 1 bis 5, **dadurch gekennzeichnet, dass** während der Stabilisierung eine automatische Aufnahme von mehreren parallel zueinander oder gemäß einem beliebigen oder vorgegebenen Muster verlaufenden und in vorgegebenen Abständen versetzten Tiefenschnittbildern erfolgt oder dass aus mehreren aufeinanderfolgenden Tiefenschnittbildern ein dreidimensionales Tiefenschnittbild erzeugt wird.

7. Verfahren nach einem der Ansprüche 1 bis 6, **dadurch gekennzeichnet, dass** die Positionen des Tiefenschnittbildes (32) und Flächenbildes (30) gespeichert werden und dass ein weiteres Tiefenschnittbild einer Verlaufsuntersuchung an genau der gleichen Stelle und mit der gleichen Ausrichtung aufgenommen wird wie bei einer vorangegangenen Untersuchung und dass die Position des weiteren Tiefenschnittbildes gespeichert wird.

8. Verfahren nach einem der Ansprüche 1 bis 7, **dadurch gekennzeichnet, dass** das zweidimensionale Flächenbild (30) über reemittiertes Licht in einem oder mehreren ausgewählten Wellenlängenbereichen erzeugt wird und/oder dass zur Aufnahme oder Erzeugung des Flächenbildes (30) die Netzhaut mit Licht eines ausgewählten Wellenlängenbereichs oder mehrerer ausgewählter Wellenlängenbereiche beleuchtet wird.

9. Verfahren nach einem der Ansprüche 1 bis 8, **dadurch gekennzeichnet, dass** die Beleuchtung zur Aufnahme des Flächenbildes (30) mit einem Punkt erfolgt, der rasterförmig einen Bereich der Netzhaut abscannt oder die Beleuchtung mit einer Linie erfolgt, welche über den Bereich der Netzhaut geführt wird.

10. Verfahren nach einem der Ansprüche 1 bis 9, **dadurch gekennzeichnet, dass** die Detektion mittels Punktdetektor oder mittels einer Zeilenkamera oder mittels einer Flächenkamera durchgeführt wird.

11. Verfahren nach einem der Ansprüche 1 bis 10, **dadurch gekennzeichnet, dass** die Aufnahme der Tiefenschnittbilder (32) nach einem Fourier Domain OCT-Verfahren oder nach einem Time Domain OCT-Verfahren erfolgt.

12. Verfahren nach einem der Ansprüche 1 bis 11, **dadurch gekennzeichnet, dass** das zweidimensionale Flächenbild (30) ein Angiographiebild ist.

13. Verfahren nach Anspruch 12, **dadurch gekennzeichnet, dass** ein erstes Angiographiebild und ein zweites zweidimensionales Netzhautbild und/oder Flächenbild gleichzeitig oder in sehr kurzem zeitlichen Abstand aufgenommen werden und dass die Kontrolle und Steuerung der Aufnahme der Tiefenschnittbilder anhand einer Aktualisierung des zweiten Flächenbildes erfolgt.

14. Verfahren, insbesondere nach Anspruch 12 oder 13, **gekennzeichnet durch** die Kombination flächiger Angiographiebilder und der insbesondere simultanen Erzeugung und/oder Darstellung der Tiefenschnittbilder (32), insbesondere OCT-Tiefenschnittbilder.

15. Vorrichtung zur Untersuchung der Netzhaut, wobei erste Daten und erste Flächenbilder der Netzhaut erzeugt werden, enthaltend eine erste optische Systemkomponente (22), welche eine Bilderfassungseinheit (4) und eine Abtaststeuereinheit (10) für die Flächenbilder enthält, wobei ferner Tiefenschnittbilder erzeugt werden, zur Durchführung des Verfahrens nach einem der Ansprüche 1 bis 14, **dadurch gekennzeichnet,**
**dass** eine zweite Systemkomponente (24) vorgesehen ist, welche eine Abtasteinheit (16) und eine Abtaststeuerung (21) zur Erzeugung der Tiefenschnittbilder enthält, und dass die Strahlengänge der beiden Systemkomponenten (22, 24) in einer gemeinsamen optischen Einheit (12) vereinigt werden, wobei die vereinigten Strahlen auf das zu untersuchende Auge gerichtet und von diesem zur optischen Einheit (12) zurückgeführt werden, mittels welcher eine entsprechende Aufteilung zur ersten Systemkomponente (22) bzw. zur zweiten Systemkomponente (24) erfolgt.

16. Vorrichtung nach Anspruch 15, **gekennzeichnet durch** eine gemeinsame Kontroll- und Verarbeitungseinheit (14) für die beiden Systemkomponenten (22,24) und dass an die Kontroll-und Verarbeitungseinheit (14) eine weitere Einheit (26), insbesondere zwecks Bedienung und Auswertung, und ferner eine Anzeigeeinheit (28) für die Flächenbilder (30) sowie die Tiefenschnittbilder (32) angeschlossen ist.

## Claims

1. Method for the examination of the retina, wherein by means of a first optical system component (22), which contains a first sampling unit (10) and an image recording unit (4), a first set of data and surface images (30) of the retina are generated, wherein
furthermore a second set of data and two-dimensional depth slice images (32) are generated from the retina, and the position of this second set of data in the surface image (30) taken of the retina is known, and/or is prescribed in accordance with the surface image, wherein furthermore by means of a second system component (24), which contains a second sampling unit (16) and a sampling control system (21), the depth slice images (32) are generated, and the location (36) and alignment (34, 35) of the depth slice image (32) is selected and prescribed on the surface image (30),
**characterised in that** the depth slice image (32) is stabilised by feedback of the eye movements measured by means of the surface image (30) to the sampling unit (16) of the second system component (24), wherein that location of the retina, at which the depth slice image is extracted, is tracked in accordance with the eye movements.

2. Method in accordance with Claim 1,
**characterised in that** the position and alignment of one or a plurality of depth slice images (32) is monitored, or actively controlled, or prescribed on the basis of the surface image (30), or a previously taken surface image.

3. Method in accordance with Claim 1 or 2,
**characterised in that** the position and alignment of one or a plurality of depth slice images (32) is prescribed on the basis of a first taken surface image (30) and is actively monitored via an update of the surface image, and/or
**in that** the movement of the updated surface image is determined relative to the first taken surface image.

4. Method in accordance with one of the Claims 1 to 3,
**characterised in that** the first surface image (30) or the updated surface image and the depth slice image (32) are represented concurrently, or simultaneously, or at prescribable, preferably small, intervals of time by means of one or a plurality of display units (28).

5. Method in accordance with one of the Claims 1 to 4,
**characterised in that** an averaging procedure is performed from a plurality of successive, preferably stabilised depth slice images, and/or
**in that** the one or more averaged depth slice images are represented in particular as a moving average value.

6. Method in accordance with one of the Claims 1 to 5,
**characterised in that** during the stabilisation procedure an automatic exposure takes place of a plurality of depth slice images, running parallel to one another or in accordance with an arbitrary or prescribed pattern, and displaced at prescribed intervals, or
**in that** a three-dimensional depth slice image is generated from a plurality of successive depth slice images.

7. Method in accordance with one of the Claims 1 to 6,
**characterised in that** the positions of the depth slice image (32) and surface image (30) are stored, and
**in that** a further depth slice image of a follow-up examination is taken at exactly the same location and with the same alignment as in a previous examination, and **in that** the position of the further depth slice image is stored.

8. Method in accordance with one of the Claims 1 to 7,
**characterised in that** the two-dimensional surface image (30) is generated via re- emitted light in one or a plurality of selected wavelength regions, and/or
**in that** for the exposure or generation of the surface image (30) the retina is illuminated with light of a selected wavelength region, or a plurality of selected wavelength regions.

9. Method in accordance with one of the Claims 1 to 8,
**characterised in that** the illumination for the exposure of the surface image (30) takes place with a point that scans a region of the retina in the form of a raster pattern, or
**in that** the illumination takes place with a line that is guided over the region of the retina.

10. Method in accordance with one of the Claims 1 to 9,
**characterised in that** the detection is performed by means of a point detector, or by means of a line camera, or by means of a surface area camera.

11. Method in accordance with one of the Claims 1 to 10,
**characterised in that** the exposure of the depth slice images (32) takes place in accordance with a Fourier domain OCT method, or in accordance with a time domain OCT method.

12. Method in accordance with one of the Claims 1 to 11,
**characterised in that** the two-dimensional surface image (30) is an angiogram.

13. Method in accordance with Claim 12,
**characterised in that** a first angiogram and a second two-dimensional retinal image and/or surface image are taken concurrently, or at very short intervals of time, and **in that** the monitoring and control of the exposure of the depth slice images takes place on the basis of an update of the second surface image.

14. Method, in particular in accordance with Claim 12 or 13,
**characterised by** the combination of planar angiograms and the in particular simultaneous generation and/or representation of the depth slice images (32), in particular OCT depth slice images.

15. Device for the examination of the retina, wherein
a first set of data and first surface images of the retina are generated, containing a first optical system component (22), which contains an image recording unit (4) and a sampling control unit (10) for the surface images, wherein
furthermore depth slice images are generated for execution of the method in accordance with one of the Claims 1 to 14,
**characterised in that** a second system component (24) is provided, which contains a sampling unit (16) and a sampling control system (21) for purposes of generating the depth slice images, and
**in that** the beam paths of the two system components (22, 24) are combined in a common optical unit (12), wherein the combined beam is directed onto the eye to be examined, and from the latter is fed back to the optical unit (12), by means of which an appropriate distribution to the first system component (22) and/or the second system component (24) takes place.

16. Device according to Claim 15,
**characterised by** a common monitoring and processing unit (14) for the two system components (22, 24), and
in that a further unit (26) is connected to the monitoring and processing unit (14), in particular for purposes of operation and evaluation, as is also a display unit (28) for the surface images (30) and the depth slice images (32) .

## Revendications

1. Procédé pour l'examen de la rétine, dans lequel un premier composant de système (22) optique comprenant une première unité de balayage (10) et une unité de détection d'image (4) permet de produire des premières données et des images de surface (30) de la rétine, dans lequel des deuxièmes données et des images en coupe transversale bidimensionnelles (32) sont en outre produites à partir de la rétine, et la position de ces deuxièmes données est connue dans l'image de surface (30) enregistrée de la rétine et/ou est indiquée conformément à l'image de surface, dans lequel les images en coupe transversale (32) sont en outre produites au moyen d'un deuxième composant de système (24) comprenant une deuxième unité de balayage (16) et une commande de balayage (21), et l'emplacement (36) et l'orientation (34, 35) de l'image en coupe transversale (32) sont sélectionnés et indiqués sur l'image de surface (30), **caractérisé en ce que** l'image en coupe transversale (32) est stabilisée sur l'unité de balayage (16) du deuxième composant de système (24), par rétro-couplage des mouvements oculaires mesurés au moyen de l'image de surface (30), dans lequel l'endroit de la rétine où est prise l'image en coupe transversale suit les mouvements oculaires.

2. Procédé selon la revendication 1, **caractérisé en ce que** la position et l'orientation d'une ou de plusieurs images en coupe transversale (32) sont contrôlées ou activement commandées ou indiquées à l'aide de l'image de surface (30) ou d'une image de surface prise antérieurement.

3. Procédé selon la revendication 1 ou 2, **caractérisé en ce que** la position et l'orientation d'une ou de plusieurs images en coupe transversale (32) sont indiquées à l'aide d'une première image de surface (30) enregistrée, et activement contrôlées par une actualisation de l'image de surface, et/ou **en ce que** le mouvement de l'image de surface actualisée est déterminé par rapport à la première image de surface enregistrée.

4. Procédé selon l'une des revendications 1 à 3, **caractérisé en ce que** la première image de surface (30) ou l'image de surface actualisée et l'image en coupe transversale (32) sont représentées en même temps ou simultanément ou par intervalles de temps prédéterminables, de préférence courts, au moyen d'une ou de plusieurs unités d'affichage (28).

5. Procédé selon l'une des revendications 1 à 4, **caractérisé en ce qu**'une moyenne est calculée à partir de plusieurs images en coupe transversale consécutives, de préférence stabilisées, et/ou **en ce que** la ou les images en coupe transversale moyennes sont représentées en particulier comme une valeur moyenne mobile.

6. Procédé selon l'une des revendications 1 à 5, **caractérisé en ce qu'**un enregistrement automatique de plusieurs images en coupe transversale, décalées selon des intervalles prédéfinis et disposées parallèlement les unes aux autres ou selon un modèle au choix ou prédéfini, est effectué pendant la stabilisation, ou **en ce qu'**une image en coupe transversale tridimensionnelle est produite à partir de plusieurs images en coupe transversale consécutives.

7. Procédé selon l'une des revendications 1 à 6, **caractérisé en ce que** les positions de l'image en coupe transversale (32) et de l'image de surface (30) sont enregistrées, et **en ce qu**'une autre image en coupe transversale d'un examen de suivi est prise exactement au même endroit et avec la même orientation que lors d'un examen précédent, et **en ce que** la position de l'autre image en coupe transversale est enregistrée.

8. Procédé selon l'une des revendications 1 à 7, **caractérisé en ce que** l'image de surface bidimensionnelle (30) est produite par une lumière réémise, dans une ou plusieurs plages de longueurs d'onde sélectionnées, et/ou **en ce que** pour l'enregistrement ou la production de l'image de surface (30), la rétine est éclairée avec la lumière d'une plage de longueurs d'onde sélectionnée ou de plusieurs plages de longueurs d'onde sélectionnées.

9. Procédé selon l'une des revendications 1 à 8, **caractérisé en ce que** l'éclairage destiné à l'enregistrement de l'image de surface (30) est effectué avec un point balayant une zone de la rétine en forme de quadrillage, ou l'éclairage est effectué avec une ligne guidée sur la zone de la rétine.

10. Procédé selon l'une des revendications 1 à 9, **caractérisé en ce que** la détection est effectuée au moyen d'un détecteur à point ou au moyen d'une caméra linéaire ou au moyen d'une caméra de surface.

11. Procédé selon l'une des revendications 1 à 10, **caractérisé en ce que** l'enregistrement des images en coupe transversale (32) est effectué selon un procédé de tomographie par cohérence optique dans le domaine de Fourier, ou selon un procédé de tomographie par cohérence optique dans le domaine temporel.

12. Procédé selon l'une des revendications 1 à 11, **caractérisé en ce que** l'image de surface (30) bidimensionnelle est une image d'angiographie.

13. Procédé selon la revendication 12, **caractérisé en ce qu**'une première image d'angiographie et une deuxième image de rétine bidimensionnelle et/ou image de surface sont enregistrées simultanément ou par intervalles de temps très courts, et **en ce que** le contrôle et la commande de l'enregistrement des images en coupe transversale sont effectués à l'aide d'une actualisation de la deuxième image de surface.

14. Procédé, en particulier selon la revendication 12 ou 13, **caractérisé par** la combinaison d'images d'angiographie surfaciques avec la production, en particulier simultanée et/ou la représentation des images en coupe transversale (32), en particulier des images en coupe transversale selon un procédé de tomographie par cohérence optique.

15. Dispositif pour l'examen de la rétine, dans lequel sont produites des premières données et des premières images de surface de la rétine, comprenant un premier composant de système (22) optique comportant une unité de détection d'image (4) et une unité de commande de balayage (10) pour les images de surface, dans lequel sont en outre produites des images en coupe transversale, pour la mise en œuvre du procédé selon l'une des revendications 1 à 14, **caractérisé en ce qu'**il est prévu un deuxième composant de système (24) comprenant une unité de balayage (16) et une commande de balayage (21) pour la production des images en coupe transversale, et **en ce que** les trajets de rayons des deux composants de système (22, 24) sont réunis dans une unité optique (12) commune, dans lequel les rayons réunis sont orientés vers l'œil à examiner, et renvoyés à partir de celui-ci vers l'unité optique (12), au moyen de laquelle est effectuée une répartition correspondante vers le premier composant de système (22) ou vers le deuxième composant de système (24).

16. Dispositif selon la revendication 15, **caractérisé par** une unité de traitement et de contrôle (14) commune pour les deux composants de système (22, 24), et en ce qu'une unité supplémentaire (26) est raccordée à l'unité de traitement et de contrôle (14), en particulier pour le fonctionnement et l'évaluation, en plus d'une unité d'affichage (28) pour les images de surface (30) ainsi que pour les images en coupe transversale (32).
